# EUROPEAN PATENT APPLICATION

(11) **EP 4 767 995 A1**
(43) Date of publication of application: **01.07.2026**
(21) Application number: 25226599.6
(22) Date of filing: 22.12.2025
(51) Int. Cl.: A61F 2/24

(54) **TRANSCATHETER DEVICE FOR TREATING TRICUSPID VALVE REGURGITATION**

(30) Priority: 31.12.2024 KR 20240201592
(71) Applicant: Tau Medical Inc., Busan, 46285 (KR)
(72) Inventor: KIM, June Hong, 48516 Busan (KR); KIM, Jin Chang, 50653 Yangsan-si (KR); PARK, Hyeon Jun, 50611 Yangsan-si (KR)
(74) Representative: FRKelly

(57) **Abstract**

The present disclosure provides a transcatheter device including: an anchor (200) anchored to an inner wall of an inferior vena cava (IVC); and a main shaft (10) having a proximal shaft (110) that extends from the anchor (200) toward a tricuspid valve (TV) along a cavotricuspid isthmus (CTI) of a heart, a middle shaft (120) that extends from the proximal shaft (110), passes through the TV, and has a spacer (300) preventing a reverse flow in the TV mounted thereon, and a distal shaft (130) that extends from the middle shaft (120) into a pulmonary artery (PA), and the distal shaft (130) of the main shaft (100) includes a first distal part (131) that extends in a curved shape having a predetermined curvature from the middle shaft (120), a second distal part (132) that extends from the first distal part (131) and bends at point B1, which is one point that is over and surrounds an aorta, toward the proximal shaft (110), and a third distal part (133) that extends from the second distal part (132) toward the proximal shaft (110).

## Description

### CROSS-REFERENCE TO RELATED APPLICATION

This application claims priority to and the benefit of Korean Patent Application No. 10-2024-0201592, filed on December 31, 2024, the disclosure of which is incorporated herein by reference in its entirety.

### BACKGROUND

### Field of the Invention

The present disclosure relates to a transcatheter device for treating tricuspid valve regurgitation.

### 2Discussion of Related Art

Tricuspid valve (TV) regurgitation in the heart is a phenomenon in which blood leaks from the right ventricle (RV) to the right atrium (RA) through the TV when the RV contracts. TV regurgitation occurs in a case in which the TV does not close properly when the RV contracts.

In recent years, placing a transcatheter device in an orifice in which the TV does not close (hereinafter referred to as "orifice") has been used as a method of treating TV regurgitation. Patent Document 1 is an application filed by the applicant of the present application, and a transcatheter device disclosed in Patent Document 1 has an anchor anchored to an inner wall of the inferior vena cava (IVC) and a main shaft that extends from the anchor and has a distal shaft disposed at a distal end and caught in the pulmonary artery (PA), and a spacer that has a predetermined volume and is disposed in the orifice of the TV is mounted on
In the transcatheter device disclosed in Patent Document 1, a middle shaft on which the spacer is mounted and the distal shaft that extends to and is caught in the PA are each formed in a curved shape having a predetermine radius. As a result, when the heart beats, a phenomenon in which the distal shaft is lifted and collides with an inner wall of the PA, that is, a lifting phenomenon, may occur. Such a lifting phenomenon may cause a problem that an impact is applied to the PA, and in particular, in a case in which the transcatheter device is mounted semi-permanently, there may be a problem that the impact toward the PA may continuously occur due to the lifting phenomenon.

Patent Document 1: International Patent Publication No. WO2024/080742

### SUMMARY OF THE INVENTION

The present disclosure is for addressing the above-described problems of the related art and is directed to providing a transcatheter device capable of suppressing a lifting phenomenon that may cause an impact to be applied to the pulmonary artery (PA).

To achieve the above objective, the present disclosure provides a transcatheter device including: an anchor anchored to an inner wall of an inferior vena cava (IVC); and a main shaft having a proximal shaft that extends from the anchor toward a tricuspid valve (TV) along a cavotricuspid isthmus (CTI) of a heart, a middle shaft that extends from the proximal shaft, passes through the TV, and has a spacer preventing a reverse flow in the TV mounted thereon, and a distal shaft that extends from the middle shaft into a pulmonary artery (PA). The distal shaft of the main shaft includes a first distal part that extends in a curved shape having a predetermined curvature from the middle shaft, a second distal part that extends from the first distal part and bends at point B1, which is one point that is over and surrounds an aorta, toward the proximal shaft, and a third distal part that extends from the second distal part toward the proximal shaft.

In the transcatheter device having the above configuration, because the distal shaft bends at point B1, which is one point that is over and surrounds the aorta, toward the proximal shaft and extends, the distal shaft is more distant from an upper wall of the PA compared to the related art. As a result, when the heart beats, the distal shaft does not collide with the upper wall of the PA even when lifted, and thus the distal shaft does not apply an impact to an inner wall of the PA.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a view showing a transcatheter device according to an embodiment of the present disclosure;
FIG. 2 is an enlarged view of a portion of a distal shaft of the transcatheter device according to an embodiment of the present disclosure;
FIG. 3 is a view showing an internal structure of a spacer in the transcatheter device according to an embodiment of the present disclosure;
FIG. 4 is a view showing a state in which the transcatheter device according to an embodiment of the present disclosure is placed on the heart;
(a) of FIG. 5 is a cross-sectional view along line A-A of FIG. 1, (b) of FIG. 5 is a cross-sectional view along line B-B of FIG. 1, and (c) of FIG. 5 is a cross-sectional view along line C-C of FIG. 1;
FIG. 6 is a view for describing a lifting phenomenon that occurs due to the conventional transcatheter device;
FIG. 7 is a view for describing a lifting suppressing effect of the transcatheter device according to an embodiment of the present disclosure;
FIG. 8 is a cross-sectional view showing a modification of an anchor in the transcatheter device according to an embodiment of the present disclosure;
FIG. 9 is a view showing a modification of an injection valve in the transcatheter device according to an embodiment of the present disclosure;
FIG. 10 is a view showing a marker provided in the transcatheter device according to an embodiment of the present disclosure;
FIG. 11 is a view showing transcatheter devices according to other embodiments of the present disclosure;
FIG. 12 is a view showing transcatheter devices according to still other embodiments of the present disclosure; and
FIG. 13 is a view showing a transcatheter device according to yet another embodiment of the present disclosure.

### DETAILED DESCRIPTION OF EXEMPLARY EMBODIMENTS

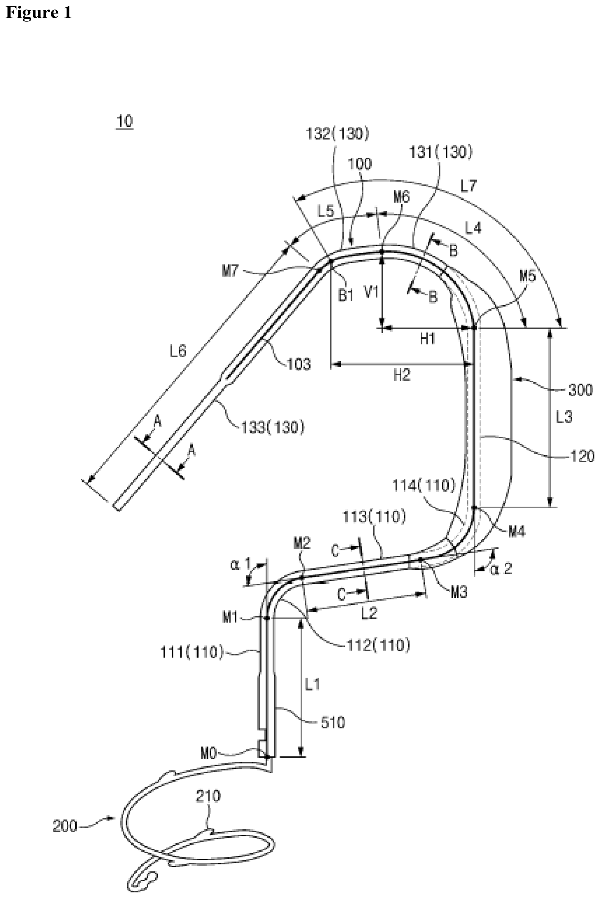

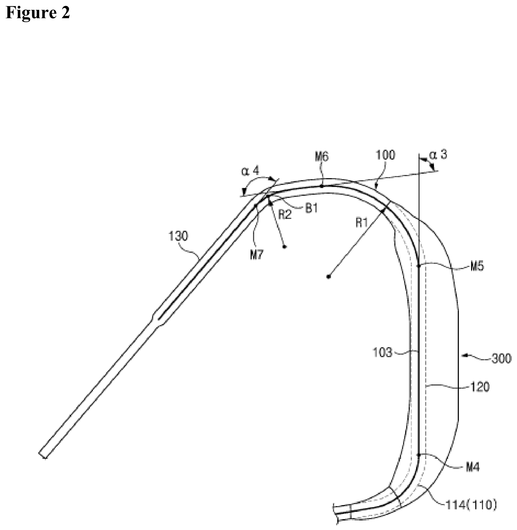

Embodiments with which the present disclosure can be implemented will be described with reference to the accompanying drawings to facilitate understanding of the present disclosure. In the drawings of this application, some components are exaggerated or omitted for convenience of description. In the following description, based on an operator, a far side is described as "distal," and a close side is described as "proximal." In addition, based on FIGS. 1 and 2, an upper side is referred to as "top," a lower side is referred to as "bottom," a left side is referred to as "left," and a right side is referred to as "right." In addition, a direction in which a main shaft and a core wire, which will be described below, extend is referred to as "extending direction." Further, in the description of the present disclosure, "bend" includes not only sharply bending at one point but also bending in a curved shape with a predetermined curvature about one point.

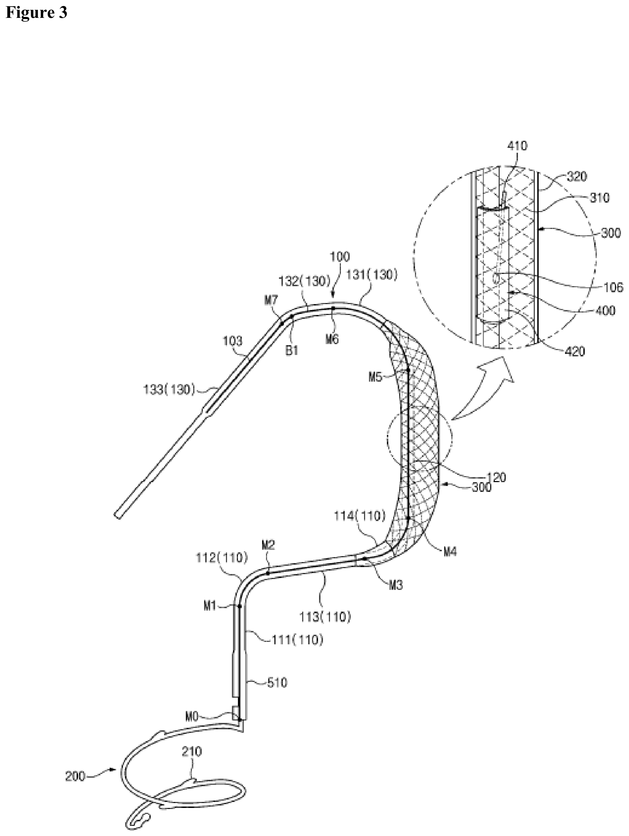

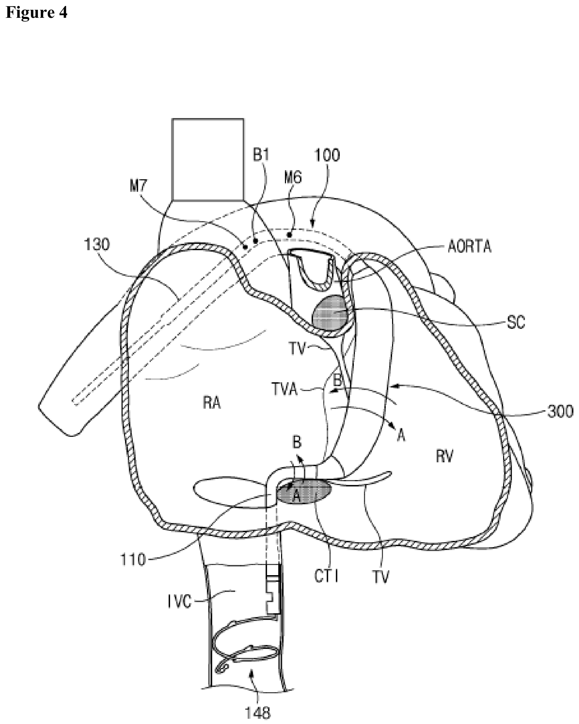

FIGS. 1 to 3 are views showing a transcatheter device 10 of the present embodiment, and FIG. 1 mainly illustrates a main shaft 100, FIG. 2 illustrates a distal shaft 130 of the main shaft 100 in an enlarged manner, and FIG. 3 mainly illustrates a spacer 300. FIG. 4 is a view showing a state in which the transcatheter device 10 according to the present embodiment is placed to obliquely cross a tricuspid valve (TV).

Referring to FIGS. 1 to 4, the transcatheter device 10 of the present embodiment has the spacer 300 disposed in an orifice of the TV to prevent a reverse flow, the main shaft 100 that places and maintains the spacer 300 to pass through the TV between an inferior vena cava (IVC) and a pulmonary artery (PA), and an anchor 200 that anchors a proximal side end of the main shaft 100 to an inner wall of the IVC.

The main shaft 100 consists of a proximal shaft 110, a middle shaft 120, and the distal shaft 130 that extend from a proximal side toward a distal side in a longitudinal direction. A core wire 103, which will be described below, is inserted into the main shaft 100. The core wire 103 maintains basic rigidity and elasticity of the main shaft 100. In the following description, points, lengths, angles, etc., used to describe each part of the main shaft 100 are based on the core wire 103.

As illustrated in FIG. 4, the proximal shaft 110 of the main shaft 100 is disposed adjacent to the IVC and is disposed adjacent to a cavotricuspid isthmus (CTI) between the IVC and the TV. The proximal shaft 110 has a substantially upside-down L-shape that generally vertically extends along the shapes of the IVC and the CTI and then generally horizontally extends, that is, "reverse L-shape." Meanwhile, such a shape of the proximal shaft 110 may be modified to various other shapes according to the structure and size of the CTI of a patient's heart.

As illustrated in FIGS. 1 to 4, the proximal shaft 110 of the present embodiment consists of a first proximal part 111 that extends from the anchor 200 anchored to the IVC (from point M0 connected to the anchor 200) upward (toward the heart) until reaching point M1, a first bent part 112 that is bent from the first proximal part 111 (from point M1 connected to the first proximal part 111) rightward toward the TV until reaching point M2, a second proximal part 113 that extends from the first bent part 112 (from point M2 connected to the first bent part 112) rightward toward the TV until reaching point M3, and a second bent part 114 that is bent from the second proximal part 113 (from point M3 connected to the second proximal part 113) upward until reaching point M4 and is connected to the middle shaft 120 at point M4. In addition, a link 510 for connecting the transcatheter device 10 and a deployment catheter (not illustrated) may be provided at a lower portion of the proximal shaft 110.

The first proximal part 111 and the second proximal part 113 of the proximal shaft 110 each have a linear shape. However, because a specific shape of the heart varies for each patient, the first proximal part 111 and the second proximal part 113 of the proximal shaft 110 extending along the shapes of the IVC and the CTI may have various other shapes according to a specific shape of the heart. For example, the first proximal part 111 and the second proximal part 113 of the proximal shaft 110 may each extend in a curved shape or may each extend in a shape in which a straight line and a curve are combined.

In the present embodiment, a length L1 of the first proximal part 111 in the extending direction (a length from point M0 to point M1) ranges approximately from 20 mm to 75 mm, and a length L2 of the second proximal part 113 in the extending direction (a length from point M2 to point M3) ranges approximately from 10 mm to 85 mm. An internal angle α1 of the first bent part 112 (an angle between a line tangent to point M1 and a line tangent to point M2) ranges approximately from 60° to 140°, and an internal angle α2 of the second bent part 114 (an angle between a line tangent to point M3 and a line tangent to point M4) ranges approximately from 60° to 140°. The length in the extending direction, which is described herein, refers to a length extending in the extending direction of the main shaft 100 (more specifically, the core wire 103), and in the case of a curve, refers to a length along a curved shape. The same applies in the following description.

In particular, in order for the first proximal part 111 to extend along the extending direction of the IVC while adhered as closely as possible to the the inner wall of the IVC and the second proximal part 113 to extend along the CTI as illustrated in FIG. 4, preferably, the anchor 200 may be installed to implement stable and firm fixing of the main shaft 100 as a whole.

Next, the middle shaft 120 is disposed to extend from point M4 connected to the second bent part 114 of the proximal shaft 110 upward toward the PA and to pass across the TV as illustrated in FIG. 4.

A length L3 of the the middle shaft 120 in the extending direction (specifically, a length from point M4 connected to the proximal shaft 110 to point M5 connected to the distal shaft 130) ranges approximately from 40 mm to 160 mm. Although the middle shaft 120 extends in a linear shape in the present embodiment, the present disclosure is not limited thereto. The middle shaft 120 may be deformed to various other shapes according to the structure and size of a patient's heart. For example, the middle shaft 120 may extend in a curved shape or may extend in a shape in which a straight line and a curve are combined.

As described above, the spacer 300 is mounted on the middle shaft 120. As illustrated in FIG. 4, the spacer 300 mounted on the middle shaft 120 is disposed in an orifice of the TV that has an end connected to a tricuspid valve annulus (TVA).

Next, as illustrated in FIG. 4, the distal shaft 130 extends from a distal side end of the middle shaft 120 toward an inner side of the PA along the PA. As illustrated in FIGS. 1 and 2, the distal shaft 130 consists of a first distal part 131 that extends in a curved shape from point M5, which is connected to the middle shaft 120, to point M6, a second distal part 132 that extends from the first distal part 131 (point M6), bends at point B1 toward the proximal shaft 110, and extends to point M7, and a third distal part 133 that extends from the second distal part 132 (point M7) to a distal side end of the distal shaft 130 toward the proximal shaft 110. Here, being bent toward the proximal shaft 110 means, when an extension line is drawn at a proximal side point, at which bending has not begun, based on point B1, being bent toward the proximal shaft 110 more than toward the extending direction of the extension line.

Preferably, the first to third distal parts 131 to 133 of the distal shaft 130 may be designed so that the distal shaft 130 has a shape along the shape of the PA, without colliding with an inner wall of the PA, as illustrated in FIG. 4 when the transcatheter device 10 is mounted on the heart. Meanwhile, the PA has a shape that is bent while surrounding the aorta at a portion connected to the heart.

In the present embodiment, as illustrated in FIGS. 1 and 2, the first distal part 131 is a portion between point M5 connected to the middle shaft 120 and point M6 connected to the second distal part 132 and may be formed of a plurality of curves having different curvatures. As illustrated in FIG. 4, point M6 is at a position that is over and surrounds the aorta.

The position of point M6 in the first distal part 131 varies according to the shape and size of the PA and the heart of a patient, but a horizontal length H1 between point M5 and point M6 ranges approximately from 20 mm to 40 mm, and a vertical length V1 between point M5 and point M6 ranges approximately from 20 mm to 50 mm. In addition, a length L4 of the first distal part 131 in the extending direction (a length from point M5 to point M6 in the extending direction) ranges approximately from 40 mm to 110 mm. An internal angle α3 of the first distal part 131 (an angle between a line tangent to point M5 and a line tangent to point M6) ranges approximately from 80° to 120°. In the present embodiment, the internal angle α3 of the first distal part 131 is approximately 90°. Due to having the internal angle α3 in the above range, the first distal part 131 extends to a position that is over and surrounds the aorta.

In addition, the second distal part 132 is a portion between point M6 connected to the first distal part 131 and point M7 connected to the third distal part 133 and is bent from point B1, which is one point of the second distal part 132, toward the proximal shaft 110. In the present embodiment, point B1, which is a bending point of the second distal part 132, is a point that is more toward the distal side than point M6 connected to the first distal part 131. That is, by point M6 being disposed at a position that is over and surrounds the aorta and point B1, which is the bending point, being disposed more toward the distal side than point M6 as illustrated in FIG. 4, the distal shaft 130 of the present embodiment is bent toward the proximal shaft 110 at the point that is over and surrounds the aorta.

When the second distal part 132 is formed in a curved shape as a whole due to a portion between point M6 and point B1 of the second distal part 132 being formed in a curved shape and a portion between point B1 and point M7 of the second distal part 132 also being formed in a curved shape, an internal angle α4 of the second distal part 132 (an angle between a line tangent to point M6 and a line tangent to point M7) ranges approximately from 90° to 130°. In the present embodiment, the internal angle α4 of the second distal part 132 is approximately 120°.

In addition, a horizontal length H2 between point B1, which is the bending point, and point M5 of the second distal part 132 ranges approximately from 30 mm to 50 mm, and a length L7 from point M5 to point B1 in the extending direction ranges approximately from 55 mm to 65 mm. In addition, a length L5 of the second distal part 132 in the extending direction (a length from point M6 to point M7 in the extending direction) ranges approximately from 10 mm to 50 mm. The above numerical ranges may vary according to the shape and size of the PA and the heart of a patient.

The portion between point M6 and point B1 of the second distal part 132 is formed in a curved shape in the present embodiment, but may also be formed in a linear shape according to the shape and size of the PA and the heart of a patient. In addition, the portion between point B1 and point M7 of the second distal part 132 is formed in a linear shape in the present embodiment, but may also be formed in a curved shape according to the shape and size of the PA and the heart of a patient.

In addition, in the present embodiment, preferably, an approximate radius of curvature R2 at point B1, which is the bending point of the second distal part 132, may be smaller than an approximate radius of curvature R1 of the first distal part 131. Here, "approximate radius of curvature" refers to, when a plurality of curves are formed or a curve and a straight line are continuously formed in combination, a radius of curvature of an approximate curve that is drawn closest thereto. For example, an approximate curve may be a curve that is drawn using the known least squares method. According to the shape and size of the PA and the heart of a patient, the approximate radius of curvature R2 at point B1, which is the bending point of the second distal part 132, ranges approximately from 13 mm to 17 mm, and the approximate radius of curvature R1 of the first distal part 131 ranges approximately from 27 mm to 33 mm.

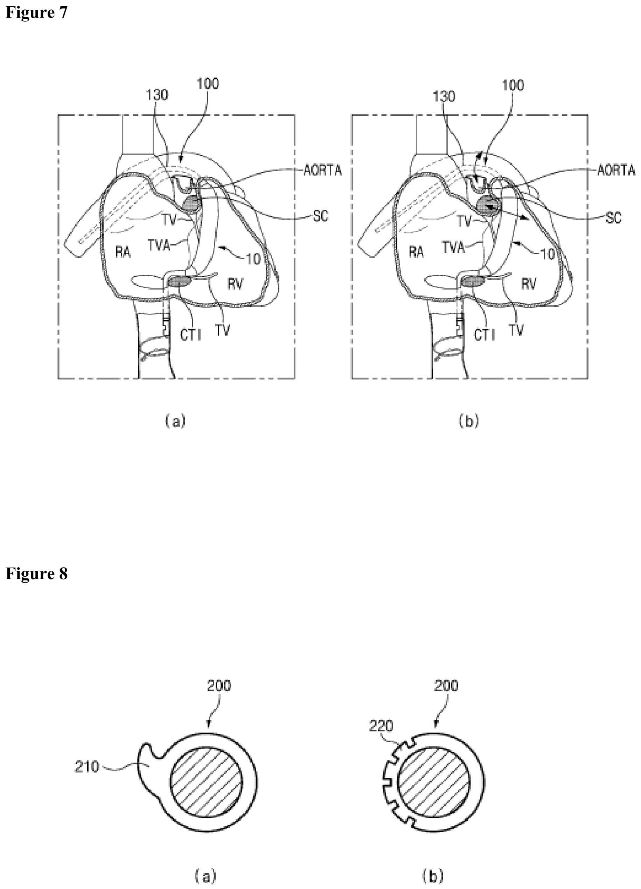

In the transcatheter device 10 of the present embodiment, because the distal shaft 130 is more bent toward the proximal shaft 110 at point B1, which is a distal side, than at point M6 that is over and surrounds the aorta, when the distal shaft 130 is placed in the PA, the distal shaft 130 is located apart from an upper wall of the PA, and even when the distal shaft 130 is lifted when the heart beats, the distal shaft 130 does not collide with the upper wall of the PA (see (a) and (b) of FIG. 7). Therefore, in the transcatheter device 10 of the present embodiment, it is possible to suppress lifting that may occur when the heart beats.

In addition, in the transcatheter device 10 of the present embodiment, because the approximate radius of curvature R2 of the second distal part 132 is smaller than the approximate radius of curvature R1 of the first distal part 131, when the distal shaft 130 is inserted into the PA, the distal shaft 130 is more reliably disposed apart from the upper wall of the PA, and even when the distal shaft 130 is lifted when the heart beats, the distal shaft 130 does not collide with the upper wall of the PA (see (a) and (b) of FIG. 7). Therefore, in the transcatheter device 10 of the present embodiment, it is possible to more reliably suppress lifting that may occur when the heart beats.

In addition, the third distal part 133 extends toward the proximal shaft 110 from point M7 connected to the second distal part 132 and may be formed in a linear shape according to the shape and size of the PA and the heart of a patient. Preferably, the third distal part 133 may extend along the shape of the PA to not come into contact with the inner wall of the PA.

In addition, the third distal part 133 may have at least one curved section according to the shape and size of the PA and the heart of a patient. However, the curve does not deviate significantly from a linear shape. An end of the third distal part 133 may be bent from a direction in which a length thereof extends. The bending range may range approximately from 140° to 200°. Although the end of the third distal part 133 may move away from the proximal shaft 110 when the end of the third distal part 133 is bent more than 180°, the lifting phenomenon does not occur. In addition, a length L6 of the third distal part 133 in the extending direction ranges approximately from 40 mm to 120.

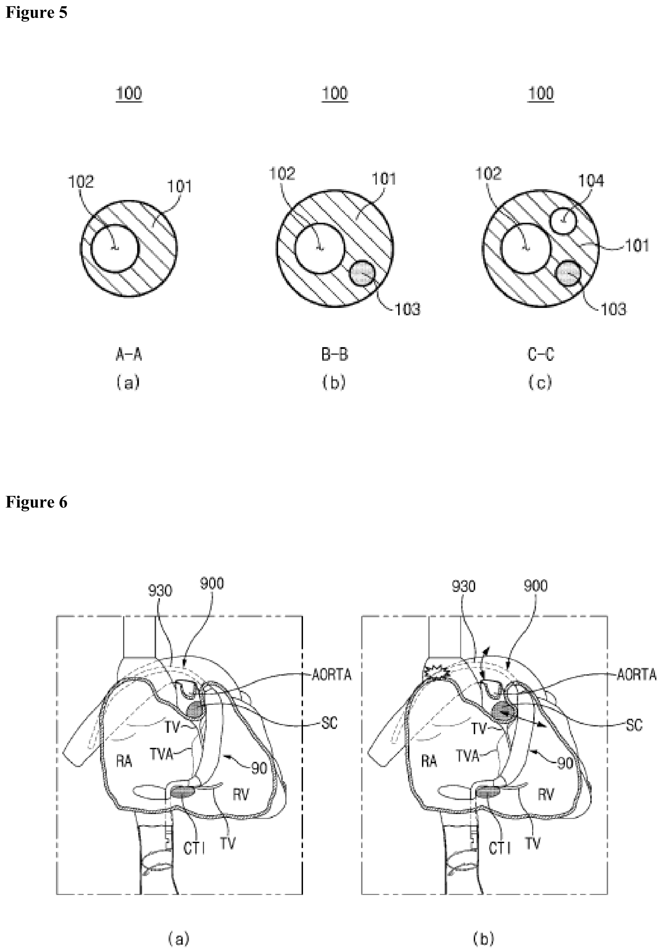

Referring to a cross-section of the main shaft 100 that is illustrated in FIG. 5, the main shaft 100 is made of a jacket 101 formed of Pellethane having a predetermined hardness. Pellethane is a brand name of a thermoplastic polyurethane elastomer and is mostly used in medical devices.

The hardness of Pellethane constituting the jacket 101 of the main shaft 100 may be different in each of the proximal shaft 110, the middle shaft 120, and the distal shaft 130 of the main shaft 100. In particular, in the distal shaft 130, the hardness of Pellethane constituting the jacket 101 may be set to progressively change toward the distal side. For example, the hardness may be set to progressively decrease toward the distal side of the distal shaft 130 to allow the distal shaft 130 to be easily deformed corresponding to the shape of the PA. In addition, the end of the third distal part 133 of the distal shaft 130 may be covered with an e-PTFE layer. The e-PTFE layer is polytetrafluoroethylene (PTFE), which is a polymer.

In addition, a guidewire lumen 102 through which a guidewire passes is formed in the jacket 101 of the main shaft 100 to guide the transcatheter device 10 during treatment throughout an entire section (see (a), (b), and (c) of FIG. 5).

In the main shaft 100, the core wire 103 is inserted into the jacket 101 in the proximal shaft 110 and the middle shaft 120 and up to a predetermined portion of the distal shaft 130 (specifically, a middle portion of the third distal part 133) (see (b) and (c) of FIG. 5). The core wire 103 may be made of a nitinol alloy or the like having a predetermined rigidity. The core wire 103 may generate elasticity that allows the overall annular shape of the main shaft 100 to be maintained and allows the main shaft 100 to return to its original shape when the main shaft 100 is deformed due to an external force.

In addition, in the main shaft 100, an injection tube lumen 104 for guiding an injection tube 410 is formed in the proximal shaft 110 and up to a predetermined vicinity of the middle shaft 120 (specifically, up to an injection hole 106 which will be described below) (see (c) of FIG. 5).

Referring back to FIGS. 1 and 2, the anchor 200 connected to the proximal shaft 110 of the main shaft 100 is made of a helical coil formed by winding a metal wire. The anchor 200 is adhered to the inner wall of the IVC by expanding. By the anchor 200 being adhered to the inner wall of the IVC and anchored, the position of the proximal shaft 110 of the transcatheter device 10 of the present embodiment is fixed.

Preferably, the anchor 200 may be anchored to the inner wall of the IVC to not move toward the heart even when the heart moves during beating. To this end, in the present embodiment, as illustrated in FIG. 1, a plurality of protrusions 210 that protrude outward and come into contact with the inner wall of the IVC in a state in which the anchor 200 is anchored to the inner wall of the IVC may be provided on an outer circumferential surface of the anchor 200. The protrusions 210 may increase friction with the inner wall of the IVC to allow the anchor 200 to be more stably anchored.

Referring to FIG. 3, the spacer 300 is provided on the middle shaft 120. The spacer 300 surrounds the middle shaft 120 and is provided across a range from one point of the second proximal part 113 of the proximal shaft 110 to one point of the first distal part 131 of the distal shaft 130.

The spacer 300 has a mesh structure 310 that surrounds the main shaft 100 and a balloon 320 that surrounds the mesh structure 310, has an inner space which remains sealed, and has a size that is adjusted according to the amount of liquid (saline solution) injected into the inner space.

The mesh structure 310 is formed by weaving wires made of a shape memory alloy or the like into a mesh shape and maintains a shape having a predetermined volume in a state in which an external force is not applied thereto. In addition, the mesh structure 310 may contract when an external force is applied thereto. One of a proximal end and a distal end of the mesh structure 310 may be fixed to the main shaft 100, and the other may move in the longitudinal direction without being fixed to the main shaft 100. Therefore, when the mesh structure 310 contracts or expands, the other that is not fixed to the main shaft 100 moves along the main shaft 100.

The balloon 320 may contract or expand due to a saline solution S filled in the inner space, and both ends of the balloon 320 are fixed to the main shaft 100. The injection hole 106 is formed in the main shaft 100 to supply the saline solution S into the balloon 320. Referring to the enlarged portion of FIG. 3, the injection hole 106 is located inside the balloon 320 and communicates with the above-described injection tube lumen 104 formed in the jacket 101 of the main shaft 100.

In addition, an injection valve 400 that opens or closes communication with the injection hole 106 is provided at an outer side of the injection hole 106. The injection valve 400 is a silicone band 420 that surrounds the injection hole 106. The injection tube 410 is inserted from the outside through the injection tube lumen 104, passes through the injection hole 106, and protrudes to the inner space of the balloon 320. Then, the silicone band 420 widens due to the injection tube 410.

In this state, the mesh structure 310 does not completely expand because the balloon 320 is contracted, but maintains a state in which a predetermined space is secured therein. Therefore, the injection tube 410 may protrude to the inner space of the balloon 320. Then, when the saline solution S is injected into the inner space of the balloon 320 through the injection tube 410, the balloon 320 expands. Accordingly, the mesh structure 310 also expands to a remembered shape.

After the balloon 320 expands to have a desired volume, when the injection tube 410 is recovered to the outside through the injection hole 106, the silicone band 420 contracts, and the injection hole 106 closes. As a result, the injected saline solution S does not exit the balloon 320, and the balloon 320 maintains the shape having a predetermined volume.

The volume of the spacer 300 having the above configuration may be adjusted according to the amount of the saline solution S injected into the balloon 320. Therefore, the size of the spacer 300 may be adjusted to fit the size or the like of an orifice in which TV regurgitation occurs. As a result, when the spacer 300 does not fit the size of an orifice during treatment, the operator only needs to adjust the amount of the injected saline solution S without recovering the spacer 300. In addition, the inside of the balloon 320 may also be filled with a material other than the saline solution S (for example, a contrast medium, a gel, a fiber, etc.).

As illustrated in FIG. 4, the transcatheter device 10 according to the present embodiment that has the above-described configuration is placed at the heart in a state in which the anchor 200 is anchored to the IVC and the distal shaft 130 is placed across the PA. At this time, the spacer 300 passes through the orifice of the TV. In this state, when the saline solution is supplied to the spacer 300 and the balloon 320 expands to a desired volume, the orifice of the TV is filled due to the balloon 320.

In a state in which the transcatheter device 10 according to the present embodiment is disposed in this way, the main shaft 100 is tilted in direction A of FIG. 4, and as a result, a force in direction A is applied to the first bent part 112 and the second bent part 114 of the proximal shaft 110. In addition, since the main shaft 100 has elasticity, an elastic force in direction B acts on the first bent part 112 and the second bent part 114 of the proximal shaft 110, and due to the elastic force in direction B, the main shaft 100 comes into contact with a supraventricular crest SC in direction B. That is, due to the elastic force in direction B of the main shaft 100, the spacer 300 mounted on the main shaft 100 is able to maintain a desired position.

Meanwhile, when a conventional transcatheter device 90 in which a distal shaft 930 is formed in a curved shape having a predetermined curvature is disposed at the heart, as illustrated in (a) of FIG. 6, the distal shaft 930 is disposed to not come into contact with the inner wall of the PA. However, as illustrated in (b) of FIG. 6, when the heart moves in directions indicated by arrows (in a 4 o'clock or 5 o'clock direction) during beating, in a portion of a main shaft 900 that comes into contact with the supraventricular crest SC, a portion at the right side of the figure (one portion of the distal shaft 930 (specifically, the portion indicated by an ellipse)) is lifted and collides with the upper wall of the PA. Because the heart reciprocates in the directions indicated by the arrows during beating, a lifting phenomenon is caused in which the distal shaft 930 repeatedly collides with the upper wall of the PA and applies an impact to the PA.

In relation to the above, in the transcatheter device 10 according to the present embodiment, the distal shaft 130 is bent toward the proximal shaft 110 at point B1 (one point that is over and surrounds the aorta), which is the bending point of the second distal part 132. As a result, in the transcatheter device 10 according to the present embodiment, as illustrated in (a) of FIG. 7, a portion ranging from point B1 to the distal side end of the distal shaft 130 is more distant from the upper wall of the PA compared to the related art. As a result, even when the heart moves in the directions indicated by the arrows (in the 4 o'clock or 5 o'clock direction) during beating and the distal shaft 130 is lifted as illustrated in (b) of FIG. 7, the distal shaft 130 does not collide with the upper wall of the PA, and as a result, the distal shaft 130 does not apply an impact to the inner wall of the PA.

In addition, in the transcatheter device 10 according to an embodiment of the present disclosure, the approximate radius of curvature R2 at point B1, which is the bending point of the second distal part 132, is smaller than the approximate radius of curvature R1 of the first distal part 131. As a result, even when the distal shaft 130 is formed in a curved shape at point B1 of the second distal part 132, the portion ranging from point B1 to the distal side end of the distal shaft 130 is reliably distant from the upper wall of the PA compared to the related art. As a result, when the heart beats, even when the distal shaft 130 is lifted as illustrated in (b) of FIG. 7, the distal shaft 130 does not collide with the upper wall of the PA, and as a result, the distal shaft 130 does not apply an impact to the inner wall of the PA.

In addition, in the transcatheter device 10 according to an embodiment of the present disclosure, because the internal angle α3 of the first distal part 131 (the angle between the line tangent to point M5 and the line tangent to point M6) ranges from 80° to 120°, point M6 is located over and surrounds the aorta, and as a result, point B1 located more toward the distal side than point M6 is reliably located at one point that is over and surrounds the aorta.

In addition, in the transcatheter device 10 according to an embodiment of the present disclosure, because the internal angle α4 of the second distal part 132 (the angle between the line tangent to point M6 and the line tangent to point M7) ranges from 90° to 130°, the portion ranging from point B1 to the distal side end of the distal shaft 130 is reliably distant from the upper wall of the PA compared to the related art.

As described above, the transcatheter device 10 according to the present embodiment can prevent the lifting phenomenon in which the distal shaft 130 applies an impact to the inner wall of the PA when the heart beats. As a result, the transcatheter device 10 according to the present embodiment can be semi-permanently placed at the heart.

### <Modifications>

Hereinafter, modifications of major components of the transcatheter device 10 according to the present embodiment will be described. FIG. 8 is a cross-sectional view showing a modification of an anchor in the transcatheter device according to an embodiment of the present disclosure. As described above, the anchor 200 is for anchoring the proximal side of the transcatheter device 10 to the IVC. In the present embodiment, a plurality of protrusions 210 are provided for the anchor 200 to increase a frictional force with the inner wall of the IVC.

As illustrated in (a) of FIG. 8, the protrusion 210 may have a cross-sectional shape that protrudes toward the heart. In this case, when the anchor 200 receives a force that draws the anchor 200 toward the heart when the heart beats, the anchor 200 can be prevented from moving toward the heart. In addition, the protrusion 210 of the present modification does not protrude to the side opposite to the heart side. As a result, when the transcatheter device 10 is recovered to the outside of the body, the anchor 200 can be easily taken out (to a lower side).

In addition, as illustrated in (b) of FIG. 8, in a state in which the anchor 200 is anchored to the inner wall of the IVC, concave and convex portions 220 that come into contact with the inner wall of the IVC may be provided in place of the protrusions 210 on the outer circumferential surface of the anchor 200. The concave and convex portions 220 may also increase a frictional force with the inner wall of the IVC when in contact with the IVC, thereby allowing the anchor 200 to be stably anchored.

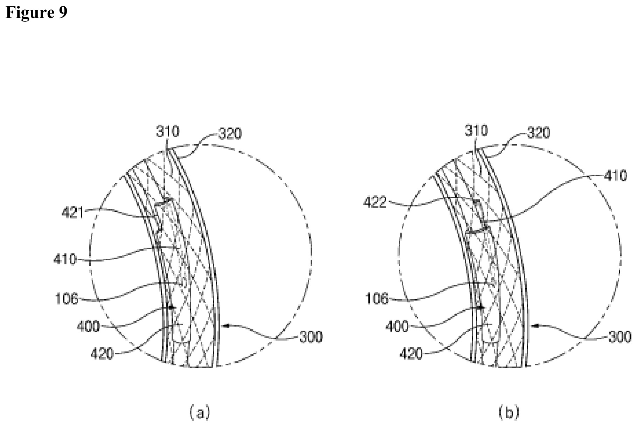

FIG. 9 is a view showing a modification of an injection valve in the transcatheter device according to an embodiment of the present disclosure. As described above, the injection valve 400 serves to open or close the injection hole 106 through which the injection tube 410 for injecting the saline solution S passes. Meanwhile, because the injection tub 410 illustrated in FIG. 3 protrudes to the inner side of the balloon 320 during injection of the saline solution S, the injection tube 410 may cause damage to the balloon 320 when in contact with the balloon 320. When the balloon 320 is damaged, the saline solution S may exit the balloon 320, and the transcatheter device 10 may not be able to perform its function.

In the present modification, as illustrated in (a) of FIG. 9, a cut-out portion 421 is formed at the distal side of the silicone band 420 to allow a gap to be formed by increasing a distance between the silicone band 420 and the main shaft 100. In addition, the injection tube 410 is located inside the silicone band 420 instead of protruding to the outside of the silicone band 420. In the present modification, even when the injection tube 410 is located inside the silicone band 420, the saline solution S can be injected into the inner space of the balloon 320 through the above gap. In addition, in the present modification, because the injection tube 410 is located inside the silicone band 420, damage to the balloon 320 due to the injection tube 410 can be blocked.

In addition, as illustrated in (b) of FIG. 9, an outer side of the injection tube 410 that protrudes to the outside of the silicone band 420 may be covered with a protective cover 422 made of Pellethane to prevent damage to the balloon 320 even when the protective cover 422 comes into contact with the balloon 320. In the present modification, the protective cover 422 is fixed to the main shaft 100 (specifically, the middle shaft 120).

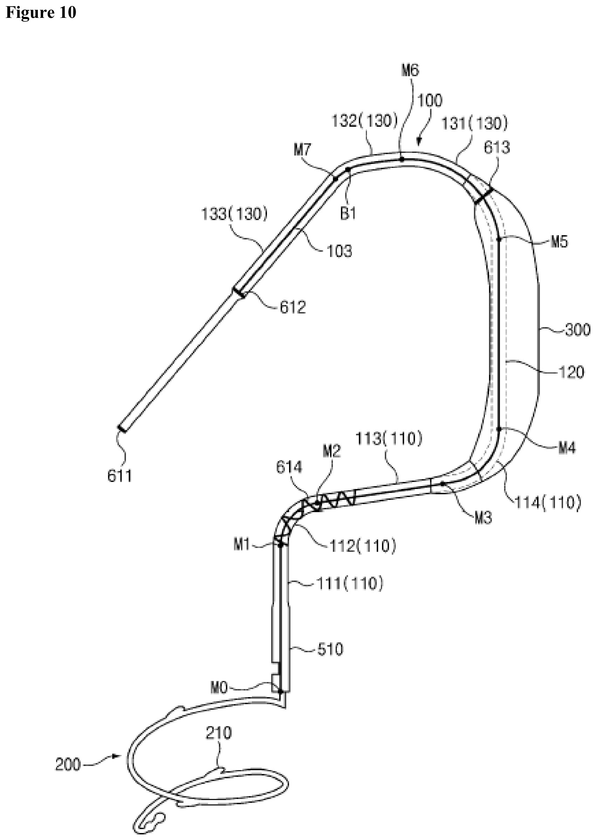

In the modification of FIG. 10, a marker for clearly identifying the position of the transcatheter device 10 during treatment is placed on the transcatheter device 10. Examples of the marker may include a marker 611 disposed on a distal side tip of the distal shaft 130, a marker 612 disposed on a front end of the core wire 103 that is a middle portion of the second distal part 132 of the distal shaft 130, a marker 613 provided in the vicinity of a distal side end of the spacer 300, and a marker 614 provided on the first bent part 112 of the proximal shaft 110.

Here, the markers 611, 612, and 613 may be band-type markers, and the marker 614 may be a strip-type marker. Through each of the markers, an operator can easily identify the position of a component of the transcatheter device 10 where the marker is located and thus can more accurately place the transcatheter device 10 at a desired position.

### <Other embodiments>

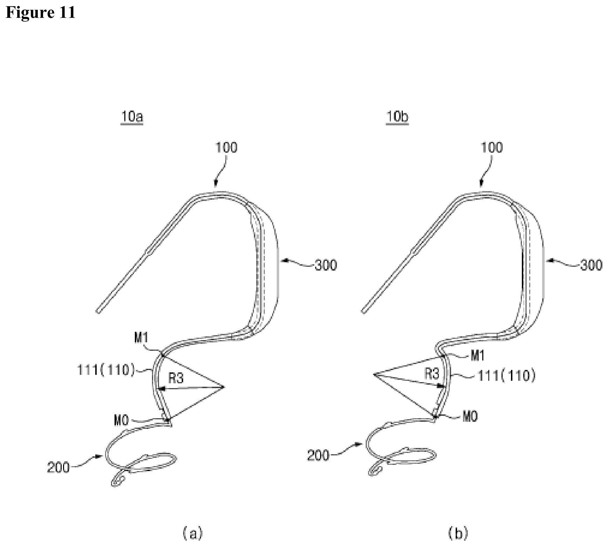

FIG. 11 is a view showing a transcatheter device 10a and a transcatheter device 10b according to other embodiments of the present disclosure. The transcatheter device 10a and the transcatheter device 10b are different from the transcatheter device 10 illustrated in FIG. 1 in terms of the shape of the first proximal part 111 of the proximal shaft 110.

In the proximal shaft 110, the first proximal part 111 may be formed along the shape of the IVC as described above, and for example, may be formed in a curved shape as illustrated in (a) of FIG. 11 and (b) of FIG. 11. In (a) of FIG. 11, the center of a radius of curvature R3 is located at the right side of the first proximal part 111, and in (b) of FIG. 11, the center of the radius of curvature R3 is located at the left side of the first proximal part 111.

In the transcatheter device 10a and the transcatheter device 10b according to the other embodiments of the present disclosure that are shown in FIG. 11, the first proximal part 111 may be formed in the shape of one curve or may be formed in a shape in which a plurality of curves or a curve and a straight line are combined. In the transcatheter device 10a and the transcatheter device 10b according to the other embodiments of the present disclosure, the approximate radius of curvature R3 of the first proximal part 111 (from point M0 to point M1) may range approximately from 90 mm to 110 mm.

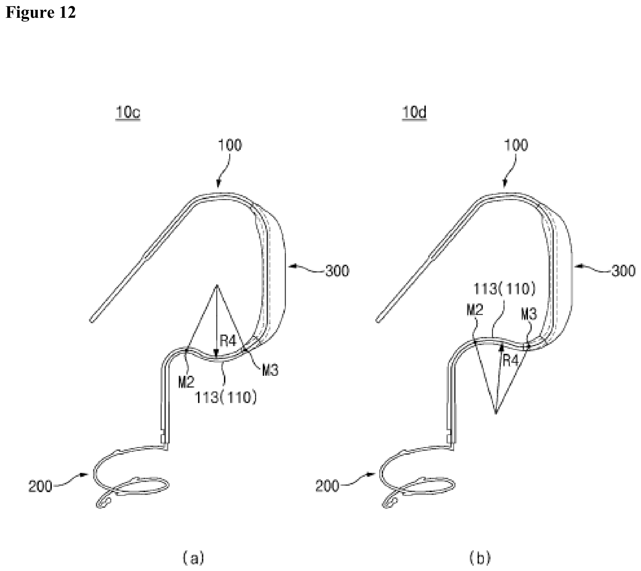

FIG. 12 is a view showing a transcatheter device 10c and a transcatheter device 10d according to still other embodiments of the present disclosure. The transcatheter device 10c and the transcatheter device 10d are different from the transcatheter device 10 illustrated in FIG. 1 in terms of the shape of the second proximal part 113 of the proximal shaft 110.

In the proximal shaft 110, the second proximal part 113 may be formed along the shape of the CTI between the IVC and the TV as described above and may be formed in a curved shape as illustrated in (a) of FIG. 12 and (b) of FIG. 12. In (a) of FIG. 12, the center of a radius of curvature R4 is located at the upper side of the second proximal part 113, and in (b) of FIG. 12, the center of the radius of curvature R4 is located at the lower side of the second proximal part 113.

In the transcatheter device 10c and the transcatheter device 10d according to the still other embodiments of the present disclosure that are shown in FIG. 12, the second proximal part 113 may be formed in the shape of one curve or may be formed in a shape in which a plurality of curves or a curve and a straight line are combined. In the transcatheter device 10c and the transcatheter device 10d according to the still other embodiments of the present disclosure, the approximate radius of curvature R4 of the second proximal part 113 (from point M2 to point M3) may range approximately from 90 mm to 110 mm.

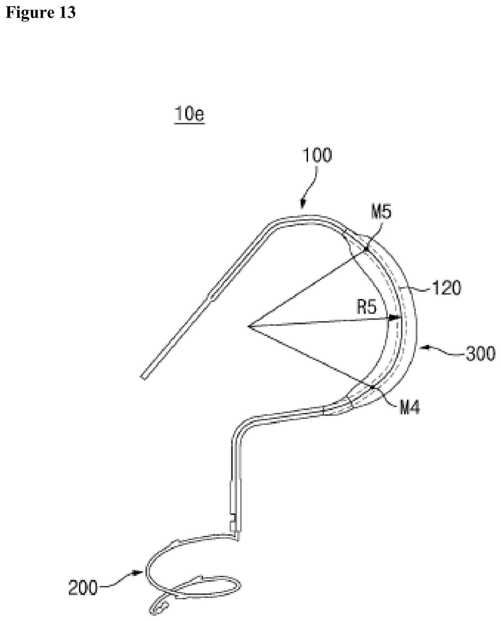

FIG. 13 is a view showing a transcatheter device 10e according to yet another embodiment of the present disclosure. Referring to FIG. 13, in the transcatheter device 10e according to yet another embodiment of the present disclosure, the middle shaft 120 is formed in a curved shape from point M4 connected to the second bent part 114 of the proximal shaft 110 to point M5 connected to the first distal part 131 of the distal shaft 130. The curved shape has a center of curvature at the left side (at the right atrium (RA) side), and a radius of curvature R5 of the middle shaft 120 (from point M4 to point M5) ranges approximately from 135 mm to 165 mm. In addition, preferably, the curved shape of the middle shaft 120 may be smoothly connected to the curved shape of the first distal part 131 of the distal shaft 130 and the curved shape of second bent part 114 of the proximal shaft 110.

A transcatheter device according to the present disclosure can suppress a lifting phenomenon that causes an impact to be applied to the PA when the heart beats.

The embodiments and modifications of the present disclosure have been described above to facilitate understanding of the gist of the present disclosure stated in the claims. Therefore, the present disclosure is not limited to these embodiments and modifications and may be modified in various ways within the scope stated in the claims. For example, the present disclosure may have a main shaft that is manufactured according to various combinations of the proximal shaft and the middle shaft disclosed in the embodiments disclosed in FIGS. 11 to 13.

## Claims

1. A transcatheter device comprising:
an anchor (200) anchored to an inner wall of an inferior vena cava (IVC); and
a main shaft (10) having a proximal shaft (110) that extends from the anchor (200) toward a tricuspid valve (TV) along a cavotricuspid isthmus (CTI) of a heart, a middle shaft (120) that extends from the proximal shaft (110), passes through the TV, and has a spacer (300) preventing a reverse flow in the TV mounted thereon, and a distal shaft (130) that extends from the middle shaft (120) into a pulmonary artery (PA),
wherein the distal shaft (130) of the main shaft (100) includes:
a first distal part (131) that extends in a curved shape having a predetermined curvature from the middle shaft (120);
a second distal part (132) that extends from the first distal part (131) and bends at point B1, which is one point that is over and surrounds an aorta, toward the proximal shaft (110); and
a third distal part (133) that extends from the second distal part (132) toward the proximal shaft (110).

2. The transcatheter device of claim 1, wherein an approximate radius of curvature (R2) at point B1, which is a bending point of the second distal part (132), is smaller than an approximate radius of curvature (R1) of the first distal part (131).

3. The transcatheter device of claim 1, wherein an internal angle (α3) of the first distal part (131) (an angle between a line tangent to point M5 connected to the middle shaft (120) and a line tangent to point M6 connected to the second distal part (132)) ranges approximately from 80° to 120°.

4. The transcatheter device of claim 1, wherein an internal angle (α4) of the second distal part (132) (an angle between a line tangent to point M6 connected to the first distal part (131) and a line tangent to point M7 connected to the third distal part (133)) ranges approximately from 90° to 130°

5. The transcatheter device of claim 1, wherein the third distal part (133) has at least one section that is bent in a curved shape toward the proximal shaft (110).

6. The transcatheter device of claim 1, wherein the middle shaft (120) has a curved shape.

7. The transcatheter device of claim 1, wherein the middle shaft (120) has a linear shape.

8. The transcatheter device of claim 1, wherein:
the proximal shaft (110) has a first proximal part (111) that extends from the anchor (200) along the IVC, a first bent part (112) that is bent from the first proximal part (111) toward the CTI of the heart, a second proximal part (113) that extends from the first bent part (112) toward the TV along the CTI of the heart, and a second bent part (114) that is bent from the second proximal part (113) and connected to the middle shaft (120); and
at least one of the first proximal part (111) and the second proximal part (113) has a curved shape.

9. The transcatheter device of any one of claims 1 to 8, wherein the anchor (200) has a helical coil shape and is anchored to the inner wall of the IVC by expanding.

10. The transcatheter device of claim 9, wherein a protrusion (210) that protrudes toward the heart and comes into contact with the inner wall of the IVC in a state in which the anchor (200) is anchored to the inner wall of the IVC is provided on an outer circumferential surface of the anchor (200) .

11. The transcatheter device of claim 9, wherein concave and convex portions (220) that come into contact with the inner wall of the IVC in a state in which the anchor (200) is anchored to the inner wall of the IVC are provided on an outer circumferential surface of the anchor (200) .

12. The transcatheter device of claim 1, wherein the spacer (300) has:
a mesh structure (310) that contracts or expands, and in a state in which an external force is not applied from surroundings, expands to have a predetermined shape; and
a balloon (320) that covers the mesh structure (310) to maintain a sealed state and has a size that is adjusted according to an amount of liquid injected into an inner space.

13. The transcatheter device of claim 12, wherein:
an injection tube lumen (104) for inserting an injection tube (410) is formed in a longitudinal direction inside the main shaft (100); and
the main shaft (100) has:
an injection hole (106) that allows communication between the injection tube lumen (104) and an inner space of the spacer (300); and
an injection valve (400) that includes a silicone band (420) that surrounds the injection hole (106) and opens or closes communication between the injection tube lumen (104) and the injection hole (106) by the injection tube (410) being inserted or withdrawn.

14. The transcatheter device of claim 13, wherein the injection tube (410) passes through the injection hole (106), and a front end of the injection tube (410) protrudes to the inner space of the spacer (300) and is surrounded by a protective cover (422).

15. The transcatheter device of claim 13, wherein a cut-out portion (421) is formed at a distal side of the silicone band (420), and the injection tube (410) passes through the injection hole (106) and has a front end located in the silicone band (420).
